(19) Europäisches Patentamt
European Patent Office
Office européen des brevets

(11) Veröffentlichungsnummer: **0 218 615**
**B1**

(12) **EUROPÄISCHE PATENTSCHRIFT**

(45) Veröffentlichungstag der Patentschrift: **12.07.89**

(51) Int. Cl.⁴: **C 07 C 127/22,** C 07 C 117/02

(21) Anmeldenummer: **86901849.9**

(22) Anmeldetag: **06.03.86**

(86) Internationale Anmeldenummer: **PCT/EP 86/00116**

(87) Internationale Veröffentlichungsnummer: **WO 86/05487 (25.09.86 Gazette 86/21)**

# (54) VERFAHREN ZUR HERSTELLUNG VON BENZOYLHARNSTOFFEN.

(30) Priorität: **14.03.85 DE 3509075**

(43) Veröffentlichungstag der Anmeldung: **22.04.87 Patentblatt 87/17**

(45) Bekanntmachung des Hinweises auf die Patenterteilung: **12.07.89 Patentblatt 89/28**

(84) Benannte Vertragsstaaten: **AT BE CH DE FR GB IT LI LU NL SE**

(56) Entgegenhaltungen:
**DE-B-1 165 587**

**Methoden der Organischen Chemie (Houben-Weyl), Band IE4, Kohlensäure-Derivate, 1983, Georg Thieme Verlag, Stuttgart (DE), sieh Seiten 763, 765**

(73) Patentinhaber: **Shell Agrar GmbH & Co. KG, Binger Strasse 173, D-6507 Ingelheim am Rhein (DE)**

(72) Erfinder: **BECHER, Heinz- Manfred, Pfarrer-Heberer- Str. 5, D-6530 Bingen am Rhein (DE)**
Erfinder: **MENGEL, Rudolf, Im Herzenacker 32, D-6535 Gau- Algesheim (DE)**
Erfinder: **OST, Walter, Pfarrer- Heberer- Str. 7, D-6530 Bingen am Rhein (DE)**

(74) Vertreter: **Hunter, Keith Roger Ian, 4 York Road, London SE1 7NA (GB)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



LIBER, STOCKHOLM 1989

**Beschreibung**

Die Erfindung betrifft ein vorteilhaftes neues Verfahren zur Herstellung von Benzoylharnstoffen, die als Schädlingsbekämpfungsmittel verwendbar sind.

Es ist bekannt Benzoylharnstoffe herzustellen, indem man entsprechende Benzoesäureamide mit substituierten Phenylisocyanaten umsetzt. Die benötigten Isocyanate werden aus den entsprechenden Anilinen durch Umsetzung mit Phosgen erzeugt. Die Aniline ihrerseits können aus den Nitroverbindungen durch Reduktion erhalten werden.

Es wurde num gefunden, dass überraschenderweise eine neue Reaktionsfolge auf technisch einfache Weise erlaubt, Benzoylharnstoffe der Formel:

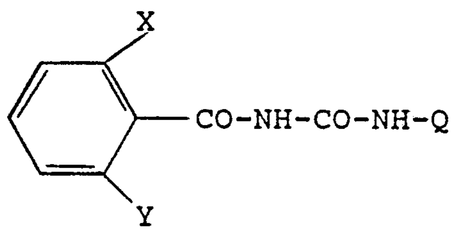

(I)

herzustellen, in der:

X für Wasserstoff, Fluor oder Chlor,
Y für Fluor, Chlor, $CH_3$ oder $CF_3$ und
Q für:
-2,3,4,5-Tetrafluorphenyl,
-2,3,4-Trifluor-5-chlorphenyl,
-2,4-Difluor-3,5-dichlorphenyl,
-3,5-Dichlor-4-pentafluorethoxyphenyl,
-3,5-Dichlor-4-[1,1,2,2-tetrafluorethoxy]-phenyl,
-3,5-Dichlor-4-trifluormethoxyphenyl,
-3,5-Dichlor-4-[(3-chlor-5-trifluormethylpyridin-2-yl)-oxyl]-phenyl,
-3-Chlor-4-[(3-Chlor-4-trifluormethyl)phenoxy]-phenyl,
-3-(4-Trifluormethylphenoxy)-phenyl,
-3-(3-Trifluormethylphenoxy)-phenyl,
-3-(2-Trifluormethylphenoxy)-phenyl,
-3,4-(Tetrafluorethylendioxy)-phenyl,
-4-Trifluormethylphenyl oder
-4-Trifluormethoxyphenyl steht.

X und Y stehen bevorzugt für die Bedeutungspaare F/H, F/F, Cl/H, Cl/Cl, Cl/F.

Die verschiedenen Substituenten können im Rahmen der obigen Definition jeweils gleich oder verschieden sein.

Das erfindungsgemässe Verfahren verläuft über die folgenden Stufen:

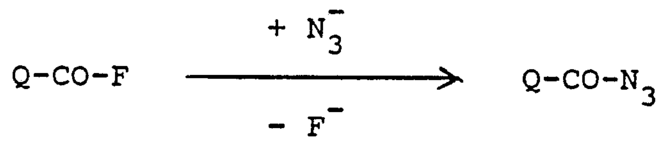


(II) (III)

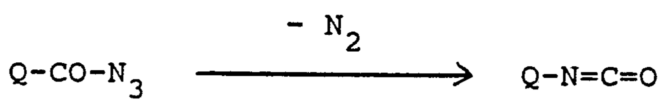


(III) (IV)

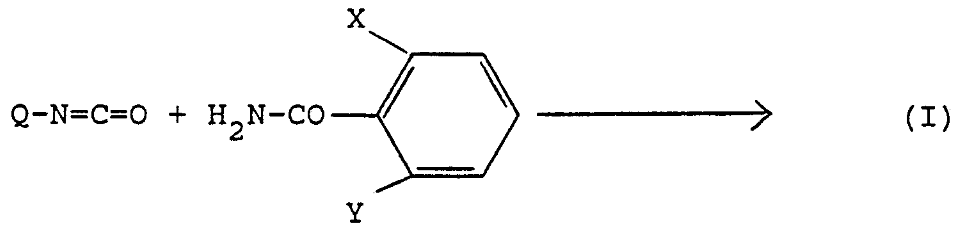

(IV) (V)

Als Azide sind Natriumazid, Kaliumazid und Calciumazid zu nennen.

Die Stufen können nacheinander in demselben Reaktionsmedium durchgeführt werden, ohne dass Zwischenprodukte isoliert werden. Die Stufen 1 und 2 werden in der Wärme, z. B. bei Temperaturen zwischen etwa 80°C und der Siedetemperatur des Reaktionsgemisches, durchgeführt, wobei Temperaturen über 180°C vermieden werden. Bevorzugt ist der Bereich zwischen etwa 100 und 140°C. Die Reaktion stellt einen thermischen Abbau der Cabonsäure über das Carbonsäureazid zum Isocyanat im Sinne des Curtius-Abbaus dar. Die Zersetzung des Azids wird so durchgeführt, dass sich jeweils keine grösseren Mengen davon im Reaktionsgemisch befinden. Als Lösungsmittel eignen sich vor allem halogenierte Benzole, etwa Chlorbenzol, dichlorbenzol, und hochsiedende Ether, ferner aromatische Kohlenwasserstoffe wie Toluol.

Die weitere Umsetzung des Isocyanats mit dem Amid V wird zwechmässig in der Weise durchgeführt, dass man das Amid V in die Lösung des Isocyanats einträgt und bei Temperaturen zwischen etwa 80 und 160°C, vorzugsweise zwischen 90° und 130°C rührt. Die Umsetzung benötigt, in Abhängigkeit von der Reaktionsfähigkeit der Komponenten und der Reaktionstemperatur, bis zu mehrere Stunden. Der erfindungsgemässe Syntheseweg über die bisher nicht beschriebenen Verbindungen III ermöglicht es, die sehr unerwünschten Umsetzungen mit Phosgen (VIII) zu vermeiden.

Ohne Zwischenisolation in den einzelnen Stufen erhält man die Verbindungen der Formel I in guter Ausbeute und Reinheit.

Als Beispiele für die Verbindungen, die erfindungsgemäss vorteilhaft hergestellt werden können, seien die folgenden Verbindungen aufgeführt:

- N-(2,4-Difluor-3,5-dichlorphenyl)-N'-(2,6-difluor-benzoyl)-harnstoff
- N-(3,5-Dichlor-4-pentafluorethoxyphenyl)-N'-(2,6-difluorbenzoyl)-harnstoff
- N-[3,5-Dichlor-4-(1,1,2,2-tetrafluorethoxy)-phenyl)-N'-(2,6-difluorbenzoyl)-harnstoff
- N-(2,3,4,5-Tetrafluorphenyl)-N'-(2,6-difluorbenzoyl)-harnstoff
- N-(2,3,4-Trifluor-5-chlorphenyl)-N'-(2,6-difluorbenzoyl)-harnstoff
- N-[3,5-Dichlor-4-(3-chlor-5-trifluormethyl-pyridin-2-yl-oxy)-phenyl]-N'-(2,6-difluorbenzoyl)-harnstoff
- N-(4-Trifluormethylphenyl)-N'-(2,6-difluor-benzoyl)-harnstoff

Hinsichtlich der Struktur-Wirkungsbeziehungen bei den insektizid wirksamen Benzoylharnstoffen ist bekannt, dass im Benzoylteil der Formel I ortho mono- oder disubstituierte Verbindungen und im Anilinteil der Formel I in meta-Stellung mono- oder disubstituierte Verbindungen besonders wirksam sind. Dabei kann im Anilinteil die ortho- und/oder die para-Stellung frei oder substituiert sein.

Konventionelle Synthesen von Benzoylharnstoffen der Formel I folgen nachstehendem Schema:

A

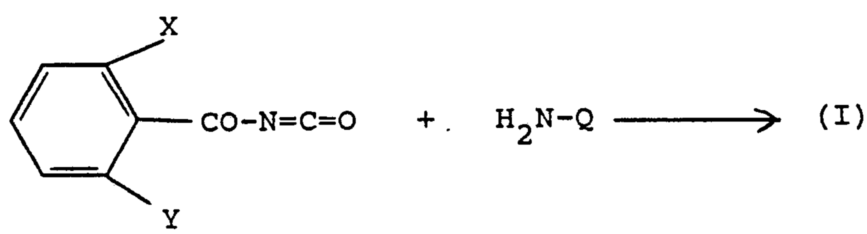

(VI) (VII)

$B_1$ Q-$NH_2$ + $Cl_2CO$ → (IV)

(VII) (VIII)

$B_2$ (V) + (IV) → (I)

Bei Reaktionsweg A - wie auch bei Reaktionsweg B der konventionellen Synthesen wird der Anilinteil entweder direkt als Anlilin (VII) oder in einem vorgeschalteten Phosgenierungsschritt $B_1$ als Isocyanat (IV) in die Synthese eingeführt.

Zum einen dirigiert die Aminogruppe des Anilins (VII) als Substituent bei dektrophilen aromatischen Substitutionsreaktionen das neu eintretende Elektrophil bevorzugt in die ortho oder para-Position. Zum anderen zeigen aber Verbindungen der Formel (I) dann besonders gute insektizide Wirkung wenn sie in der meta-Position des Anilinteils einen oder mehrere (vorzugsweise Halogen-) Substituenten tragen. Dies führt dazu, dass zur Darstellung der Anilin (VII) nicht vom Anilin oder anderen in der meta-Position unsubstituierten Anilinen ausgegangen werden kann, sondern vielmehr ein Umweg über das Nitrobenzol eingeschlagen werden muss in dem unter Ausnutzung der meta-dirigierenden Wirkung der Nitrogruppe die Substituenten in die meta-Position eingeführt werden. Die Nitroverbindung wird anschliessend Verfahren von einem Benzoesäurederivat der Formel (II) aus, bei dem die Carbonsäurefunktion in (II) als meta dirigierender Substituent in vorteilhafter Weise auch zum Aufbau des Substitutionsmuster in dem Rest Q mittels nucelophiler und elektrophiler Substitutionsreaktionen im Benzolring verwendet werden kann:

- Zum Aufbau der aus der DE-OS-3 044 055 bekannten 2,4-Difluor-3,5-dichlorphenylsubstitution aus 2,3,4,5-Tetrachlorbenzoylchlorid mit Fluorid-Ionen zum 2,4-Difluor-3,5-dichlorbenzoylfluorid etwa analog EP-A-164 619.

Bei den notwendigen Chlor-Fluor-Austauschreaktionen, wenn man von Benzoylchloriden ausgeht, neben dem Halogenaustausch im Kern des Aromaten auch das halogen der Säurehalogenidfunktion ausgetauscht, wobei dass das so erhaltene Benzoylfluorid (II) mit Aziden in der Reaktionsstufe 1 schneller und vollständiger reagiert als das entsprechende Säurechlorid.

**Beispiel 1**

N-(2,3,4-Trifluor-5-chlorphenyl)-N'-(2,6-difluorbenzoyl)-harnstoff

1a) In einem Dreihalskolben werden 0,62 g Natriumazid in 30 ml Chlorbenzol unter Rühren auf 120° C erhitzt. Bei dieser Temperatur wird zunächst ein Drittel der Lösung von 1,9 g 2,3,4-Trifluor-5-chlorbenzoylfluorid in 10 ml Chlorbenzol zugetropft. Nach einigen Minuten, wenn die Stickstoffentwicklung eingesetzt hat, wird innerhalb von 10 Minuten der Rest zugetropft. Während dieser Zeit wird die Stickstoffentwicklung lebhaft. Wenn sie aufgehört hat, läßt man noch 1/2 Stunde nachreagieren. Nach Abkühlen auf 25° C wird 0,5 g Kieselgur zugesetzt und die Mischung abgesaugt. Das Filtrat, das eine Lösung von 2,3,4-Trifluor-5-chlorphenylisocyanat darstellt, wird für die nächste Reaktionsstufe eingesetzt.

1b) Das Filtrat der vorhergehenden Reaktion wird mit 1,42 g 2,6-Difluorbenzamid (3 Stunden bei 60° C im Vakuum getrocknet) versetzt und unter Rühren aufgeheizt, bis eine klare Lösung entstanden ist (ca. 100° C). Dann wird 7 Stunden bei 100° C gerührt. Man läßt dann auf 70° C abkühlen und engt im Vakuum zur Trockne ein. Der Rückstand wird mit 30 ml Aceton bei 40 - 50° C verrührt, auf 10° C abgekühlt, abgesaugt und getrocknet.

Ausbeute: 2,1 g (64 % d. Th.); Fp 222 - 224° C.

1c) Die Vorstufe, 2,3,4-Trifluor-5-chlorbenzoylfluorid (Kp 67 mbar: 97 - 100° C) wird neben 2,4-Difluor-3,5-dichlorbenzoylfluorid (Kp 67 mbar: 127 - 129°C) erhalten, wenn man 41,7 g 2,3,4,5-Tetrachlorbenzoylchlorid mit 43,5 g Kaliumfluorid in 450 ml Sulfolan 8 Stunden auf 200°C erhitzt und das Reaktionsgemisch im Vakuum destilliert.

**Beispiel 2**

N-(2,4-Difluor-3,5-dichlorphenyl)-N'-(2,6-difluorbenzoyl)-harnstoff

2a) -2,4-Difluor-3,5-dichlorbenzoylfluorid Analog zu der in Beispiel 1b beschriebenen Umsetzung erhält man die Titelverbindung in über 70 % Ausbeute durch Umsetzung von 2,3,4,5-Tetrachlorbenzoylchlorid mit Kaliumfluorid in Sulfolan. Die Temperatur beträgt 150°C, die Reaktionszeit 8 Stunden.

2b) Umsetzung von 2,4-Difluor-3,5-dichlorbenzoylfluorid mit Natriumazid

2,4-Difluor-3,5-dichlorbenzoylfluorid (11,45 g) werden zu einer auf etwa 110°C erwärmten Suspension von Natriumazid (3,25 g) in Toluol (40 ml) getropft, wobei der Reaktionsverlauf durch das freiwerdende Stickstoffgas kontrolliert wird. Nach Beendigung der Zugabe des Säurefluorids wird noch eine Stunde bei 110°C gerührt, wobei sich Natriumfluoridkristalle niederschlagen.

2c) Umsetzung von 2,4-Difluor-3,4-dichlorphenylisocyanat mit 2,6-Difluorbenzamid

Zu der nach Beispiel 2b erhaltenen Suspension wird 2,6-Difluorbenzamid (7,9 g) gegeben und das Reaktionsgemisch dann 10 Stunden bei etwa 95°C belassen, wobei sich im Reaktionsverlauf ein weißer Kristallbrei abscheidet.

Nach dem Erhalten wird vom Niederschlag abgesaugt. Man isoliert 18,45 g eines Gemisches von Natriumfluorid und Benzoylharnstoff. Das so erhaltene Rohprodukt wird in Aceton aufgekocht. Man isoliert aus dem Filtrat 16,2 g N-(2,4-Difluor-3,5-dichlorphenyl)-N'-(2,6-Difluorbenzoyl)-harnstoff (84,36 % d. Th.).

**Beispiel 3**

N-(2,4-Difluor-3,5-dichlorphenyl)-N'-(2,6-difluorbenzoyl)-harnstoff

In die 125°C heiße Mischung von 7,0 g (0,105 mol) Natriumazid in 300 ml Chlorbenzol wird unter gutem Rühren die Lösung von 23 g (0,100 mol) 2,4-Difluor-3,5-dichlorbenzoylfluorid in 100 ml Chlorbenzol langsam eingetropft. Dabei wird Stickstoff frei. Die Zugabe wird aus Sicherheitsgründen so geregelt, daß die Menge des eingetropften Benzoylfluorids der (an dem freiwerdenden Stickstoff meßbaren) Umlagerungsrate des Curtius-Abbaus entspricht.

Nach beendeter Zugabe läßt man noch ca. 30 Minuten nachreagieren, gibt 3 g Kieselgur zu, läßt auf ca. 30°C abkühlen und saugt das Ungelöste ab.

Zum Filtrat gibt man 15,7 g (0,10 mol) 2,6-Difluorbenzamid und erhitzt ca. 7 Stunden auf 100°C. Die Aufarbeitung erfolgt wie in Beispiel 1 beschrieben.

Ausbaute: 28,8 g (75,3 % d. Th.); Fp. 221 - 224°C

**Patentansprüche**

1. Verfahren zur Herstellung von Verbindungen der Formel:

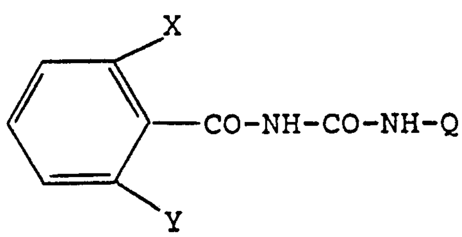

(I)

in der:

X für Wasserstoff, Fluor oder Chlor

Y für Fluor, Chlor, $CH_3$ oder $CF_3$ und

Q für:

-2,3,4,5-Tetrafluorphenyl,

-2,3,4-Trifluor-5-chlorphenyl,
-2,4-Difluor-3,5-dichlorphenyl,
-3,5-Dichlor-4-pentafluorethoxyphenyl,
-3,5-Dichlor-4-[1,1,2,2-tetrafluorethoxy]-phenyl,
-3,5-Dichlor-4-trifluormethoxyphenyl,
-3,5-Dichlor-4-[(3-chlor-5-trifluormethylpyridin-2-yl)-oxyl]-phenyl,
-3-Chlor-4-[(3-Chlor-4-trifluormethyl)-phenoxy]-phenyl,
-3-(4-Trifluormethylphenoxy)-phenyl,
-3-(3-Trifluormethylphenoxy)-phenyl,
-3-(2-Trifluormethylphenoxy)-phenyl,
-3,4-(Tetrafluorethylendioxy)-phenyl,
-4-Trifluormethylphenyl oder
-4-Trifluormethoxyphenyl steht,
dadurch gekennzeichnet, dass man ein Benzoylderivat der Formel:

Q - COF (II)

in der Q die obige Bedeutung hat
in einem geeigneten inerten Lösungsmittel in der Wärme mit einem Alkaliazid oder Erdalkaliazid umsetzt, das erhaltene Azid der Formel:

$Q - CON_3$ (III)

in Reaktionsgemisch thermisch zum Isocyanat der Formel:

Q - NCO (IV)
abbaut und dieses mit geeigneten Benzamiden der Formel:

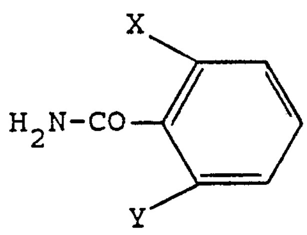

(V)

im Reaktionsgemisch unter Erwärmen zum Endprodukt der Formel I umsetzt.

2. Verfahren nach Anspruch 1, dadurch gekennzeichnet, dass man als Lösungsmittel Chlorbenzol oder Toluol benutzt.

3. Verfahren nach Anspruch 1, dadurch gekennzeichnet, dass man als Alkaliazid Natriumazid verwendet.

4. Verfahren nach Anspruch 1 bis 3, dadurch gekennzeichnet, dass man als Säurefluorid 2,4-Difluor-3,5-dichlorbenzoylfluorid und als Benzamid der Formel V 2,6-Difluorbenzamid verwendet.

**Claims**

1. Process for the preparation of compounds of the formula:

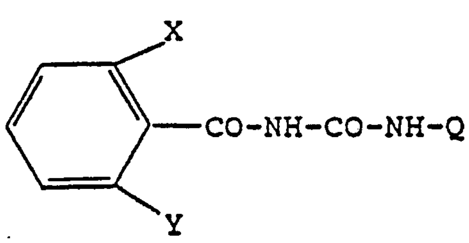

(I)

wherein:
X represents hydrogen, fluorine or chlorine
Y represents fluorine, chlorine, $CH_3$ or $CF_3$ and
Q represents:

-2,3,4,5-tetrafluorophenyl,
-2,3,4-trifluoro-5-chlorophenyl,
-2,4-difluoro-3,5 dichlorophenyl,
-3,5-dichloro-4-pentafluoroethoxyphenyl,
-3,5-dichloro-4-[1,1,2,2-tetrafluoroethoxy]-phenyl,
-3,5-dichloro-4-trifluoromethoxyphenyl,
-3,5-dichloro-4-[(3-chloro-5-trifluoromethylpyridine-2-yl)-oxyl]-phenyl,
-3-chloro-4-[(3-chloro-4-trifluoromethyl)-phenoxy]-phenyl,
-3-(4-trifluoromethylphenoxy)-phenyl,
-3-(3-trifluoromethylphenoxy)-phenyl,
-3-(2-trifluoromethylphenoxy)-phenyl,
-3,4-(tetrafluoroethylenedioxy)-phenyl,
-4-trifluoromethylphenyl or
-4-trifluoromethoxyphenyl,

characterized in that a benzoyl derivative of the formula:

Q - COF (II)

wherein:

Q has the meaning given above, is reacted under heat in a suitable inert solvent with an alkaline azide or alkaline earth azide, the resultant azide of the formula:

Q - $CON_3$ (III)

is converted thermally in a reaction mixture to isocyanate of the formula:

Q - NCO (IV)

and this is reacted in a reaction mixture while heating with suitable benzamides of the formula

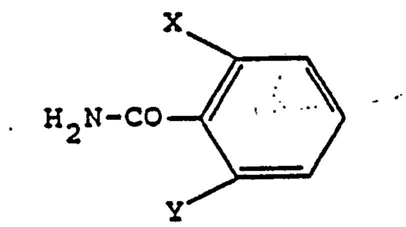

(V)

producing the end product of formula I.

2. Process according to claim 1, characterized in that chlorobenzene or toluene are used as a solvent.

3. Process according to claim 1, characterized in that sodium azide is used as an alkaline azide.

4. Process according to claims 1 to 3, characterized in that 2,4-difluoro-3,5-dichlorobenzoyl fluoride is used as an acid fluoride and 2,6-difluorobenzamide is used as a benzamide of formula V.

**Revendications**

1. Procédé pour préparer des composés de formule:

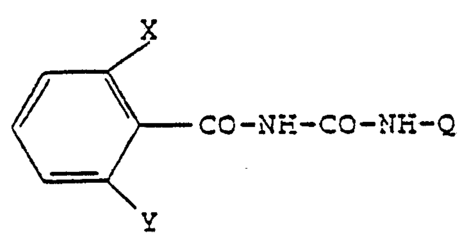

(I)

dans laquelle:

X représente l'hydrogène, le fluor ou le chlore

Y représente le fluor, le chlore, $CH_3$ ou $CF_3$ et

Q représente:

- le 2,3,4,5-tétrafluorophényle,

- le 2,3,4-trifluoro-5-chlorophényle,
- le 2,4-difluoro-3,5-dichlorophényle,
- le 3,5-dichloro-4-pentafluoroéthoxyphényle,
- le 3,5-dichloro-4-[1,1,2,2-tétrafluoréthoxy]-phényle,
- le 3,5-dichloro-4-trifluorométhoxyphényle,
- le 3,5-dichloro-4-[(3-chloro-5-trifluorométhyl-pyridine-2-yl)-oxy]-phényle,
- le 3-chloro-4-[(3-chloro-4-trifluorométhyl)-phénoxy]-phényle,
- le 3-(4-trifluorométhylphénoxy)-phényle,
- le 3-(3-trifluorométhylphénoxy)-phényle,
- le 3-(2-trifluorométhylphénoxy)-phényle,
- le 3,4-(tétrafluoroéthylènedioxy)-phényle,
- le 4-trifluorométhylphényle ou
- le 4-trifluorométhoxyphényle,

caractérisé en ce que l'on transforme un dérivé benzoyle de formule:

Q - COF (II)

dans laquelle:

Q a la signification précédente dans un solvent adepté inerte, à chaud, avec un azoture alcalin ou un azoture alcalinoterreux, en ce que l'on décompose l'azide obtenu de formule:

$Q - CON_3$ (III)

par voie thermique, dans le mélange réactionnel, en l'isocyanate de formule:

Q - NCO (IV)

et en ce que l'on transforme celui-ci avec un benzamide approprié de formule:

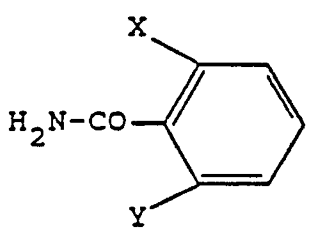

(V)

dans le mélange réactionnel, par chauffage, pour obtenir le produit final de formule I.

2. Procédé selon la revendication 1, caractérisé en ce que l'on utilise comme solvant le chlorobenzène ou le toluène.

3. Procédé selon la revendication 1, caractérisé en ce que l'on utilise comme azoture alcalin l'azoture de sodium.

4. Procédé selon l'une quelconque des revendications 1 à 3, caractérisé en ce que l'on utilise comme fluorure d'acide le fluorure de 2,4-difluoro-3,5-dichlorobenzoyle et comme benzamide de formule V le 2,6-difluorobenzamide.